# EUROPEAN PATENT SPECIFICATION

(11) **EP 4 333 951 B1**
(45) Date of publication and mention of the grant of the patent: **11.12.2024**
(21) Application number: 22727892.6
(22) Date of filing: 06.05.2022
(51) Int. Cl.: A61M 15/00

(54) **UNIT DOSE DRY POWDER INHALER**
EINZELDOSISTROCKENPULVERINHALATOR
INHALATEUR DE POUDRE SÈCHE À DOSE UNITAIRE

(30) Priority: 07.05.2021 EP 21172658
(43) Date of publication of application: 13.03.2024
(73) Proprietor: Vectura Delivery Devices Limited, Chippenham, Wiltshire SN14 6FH (GB)
(72) Inventor: MELINIOTIS, Andreas, Cambridge Cambridgeshire CB4 0GZ (GB); BAYLISS, Justin, Cambridge Cambridgeshire CB4 0GZ (GB)
(74) Representative: Clarke, Christopher John
(86) International application number: PCT/EP2022/062245
(87) International publication number: WO 2022/234065

(56) References cited:
- WO-A2-03/103563
- DE-A1- 102014 017 409
- US-A1- 2008 135 441
- US-A1- 2013 291 865
- US-A1- 2017 119 982
- US-A1- 2019 358 414
- US-B2- 10 124 129

## Description

### Technical Field of the invention

The present invention relates to an inhalation device for oral or nasal delivery of medicament in powdered form, more specifically to a unit dose inhaler which is formed as a blister that contains a dose of medicament for inhalation.

### Background to the invention

Dry powder formulations for inhalation are commonly pre-packaged in individual doses, usually in the form of capsules or blisters. A blister is typically cold-formed from a ductile foil laminate and has a puncturable or peelable lid. The lid is usually heat-sealed around the periphery of the blister after the dose of powder has been placed into the blister. Multi-dose inhalers, such as those disclosed in WO 2005/037353, contain a blister strip with number of doses to be used over a period of time, so that there is no need to insert a blister into the device each time it is used. There are also unit-dose devices that receive only one blister at a time, for example as disclosed in WO 2010/086285. Once the dose contained in a blister has been inhaled, the blister is removed from the device and discarded by the user. A new blister is then inserted for a subsequent dose.

Single dose, disposable, blister-like dispensers are also known. WO 2014/175815 discloses an inhaler with a body comprising an air channel that contains a powder and a foil with inlet and outlet holes that are closed by a removable tape. The air outlet is simply a hole in the lid, which is not very convenient for the user to inhale on. WO 2003/103563 discloses a unit dose powder inhaler with a lower wall formed from a sheet element and a peel-off piece of lidding material, such as foil. The lower wall has a recess that contains the medicament and a channel. To use the inhaler, the user breaks off a corner piece along a fracture line, which opens an air outlet at the end of the channel. An air inlet is formed by peeling off a portion of the lidding material. Alternatively, the lower wall has a further channel extending from the recess to the opposite corner, and the air inlet hole is formed by breaking off this corner. The user then inhales on the air outlet, which aerosolizes the powder. However, in this inhaler, some of the powder could transfer into the corner piece before use, and hence be lost when it is broken off, so that the full dose is not delivered to the user. DE102014017409 discloses a single-dose powder inhaler composed of a housing part in which a medicament chamber is formed and a covering element.

US2017/0119982 discloses an inhaler in which the body of the inhaler is held in a blister. The lid of the blister is connected to a seal which is located between a dose chamber and the airway formed in the body of the inhaler. The seal ensures that the powder is not transferred out of the dose chamber into the body or the blister (from which it might not be aerosolized), e.g. during transport or storage. When the lid is removed, the seal is pulled away so that the dose chamber is opened and the inhaler is thereby prepared for use. However, to achieve this, the inhaler is constructed from five separate parts: the base and lid of the blister, the body, the removable seal and the dose chamber.

US 2013/0291865 discloses a disposable unit dose inhaler with a housing having a lid, a dose chamber which contains the powder for inhalation and a dedicated mouthpiece (e.g. a tube) which is movable (rotatable or slidable) relative to the housing. When the user moves the mouthpiece relative to the housing, an opening is created and the dose chamber is opened, so that the mouthpiece is exposed for the user to inhale on, thereby causing the powder to be aerosolized. The dose chamber ensures that the powder is not transferred to another part of the housing (from which it might not be aerosolized), e.g. during transport or storage. Since the powder is held in the dose chamber, a consistent and predictable dose is delivered. However, the device is relatively complex to produce. Firstly the mouthpiece must be securely attached to the housing (so that it cannot come loose and be lost or even swallowed) in a way that permits the rotational or sliding movement. Secondly the dose chamber must be sealed before use, and then opened by the movement of the mouthpiece. In one embodiment, the mouthpiece is attached to the housing by a living hinge and a foil barrier forms the dose chamber which is pierced when the mouthpiece is moved. In another embodiment, the mouthpiece is mounted on a separate base. These co-operate to form the dose chamber, which is opened when the mouthpiece is rotated relative to the base. These require additional components (such as the barrier foil and the base) and additional process steps to manufacture the inhaler, which increases the costs and complexity.

Thus there remains a need for a unit dose inhaler that addresses these drawbacks, and which is simple and cost-effective to manufacture.

### Brief Description of the invention

The present invention provides a very simple, low cost a unit-dose dry powder inhaler which is easy to use and inexpensive to produce, because its manufacture is based on the conventional blister strip production process. Accordingly, in a first aspect, the present invention provides a unit dose dispenser in the form of a blister containing a dry powder for inhalation, wherein the dispenser comprises:
- a base in which a cavity is formed, wherein the cavity comprises a bowl that contains the powder and a channel that opens into the bowl, and has an end remote from the bowl;
- a lid, such as a foil or foil laminate, which is sealed to the base around the cavity;
- an airway located in the channel, the airway comprising a mouthpiece and a body portion having at least one air outlet passage, at least one air inlet passage, a lower surface, and an upper surface, wherein:
   - each air outlet passage opens into the bowl at a proximal end and extends from the body portion through the mouthpiece to an air outlet at a distal end;
   - each air inlet passage opens into the bowl at a proximal end and has an air inlet at a distal end;
   - the air inlet(s) and the air outlet(s) abut, and are closed by, the base and / or the lid;
   - the lower surface of the body portion matches the shape of the channel so that it forms an interference fit with the channel;
   - the upper surface of the body portion is flat and level with the base around the cavity, and the lid is preferably sealed to part of the upper surface;
- wherein part of the base and the lid of the dispenser is detachable, so that the air inlet(s) and the air outlet(s) are opened when the detachable part is removed, while the airway is retained in the channel.

The fact that the air inlet(s) and the air outlet(s) abut, and are closed by, the base and/or the lid ensures that the whole dose is retained within the airway. Since powder cannot leave the airway, little or no powder is lost when the detachable part is removed, so the whole dose is inhaled. Consequently, in contrast to the devices of US2017/0119982 and US 2013/0291865, there is no need for a separate way of sealing the powder compartment, so that additional components and process steps for securing the airway to the housing and for forming a closed dose chamber are not required.

The body portion of the airway and the channel may be semi-circular in cross-section.

The air outlet(s) may match the shape of the end of the channel so that the air outlet(s) abut, and are closed by, the end of the channel. For example, the air outlet may be curved to match the shape of the end of the channel. The air outlet(s) may be formed in the upper surface of the mouthpiece so that they abut, and are closed by, the lid.

The airway may comprise a barrier located between the air inlet(s) and the air outlet(s), wherein the size and shape of the barrier corresponds to the cross-section of the channel. The barrier may be a wall, or it may be extended to form a block. The block may be solid, or it may be hollow, apart from the wall. The lid is preferably not sealed to the upper surface of the block.

The lower surface of the body portion may extend continuously to the barrier so that the air inlets are formed in the upper surface of the airway between the upper surface of the body portion and the barrier so that they abut, and are closed by the lid.

The air inlet and / or air outlet passages may have baffles or corners or be formed as a labyrinth.

The airway may have one air outlet passage. The air outlet passage may have an extension that protrudes into the bowl. The end of the extension may be located at, or close to, the centre of the bowl. The extension may taper so that the end is narrow.

The airway may have one air outlet passage and one air inlet passage, so that the air flow is asymmetric and creates a cyclone in the bowl.

The airway may have one air outlet passage and two air inlet passages. The air inlet passages may be on either side of the air outlet passage. The air inlet passages may have extensions that protrude into the bowl.

The dispenser may have a line of weakness, such as perforations, in the base and / or the lid. The dispenser may have a notch in one or both edges of the base and / or the lid. The line of weakness and the notches may facilitate removal of the detachable part.

A pair of dispensers may be joined together so that the powders in both dispensers can be inhaled simultaneously. A plurality of dispensers may be joined together in the form of a strip which provides a multi-day supply of powder, wherein each dispenser is detachable from the rest of the strip.

In a second aspect, the invention provides a process for producing unit dose dispensers, in particular dispensers according to the first aspect of the invention, the process comprising:
a) forming cavities in a base material, each cavity comprising a bowl, and a channel that opens into the bowl and has an end remote from the bowl;
b) simultaneously or in either order, filling the powder into the bowls and placing an airway into the channel, the airway comprising a mouthpiece and a body portion having at least one air outlet passage, at least one air inlet passage, a lower surface, and an upper surface, wherein:
   - each air outlet passage opens into the bowl at a proximal end and extends from the body portion through the mouthpiece to an air outlet at a distal end; and
   - each air inlet passage opens into the bowl at a proximal end and has an air inlet at a distal end;
   - the lower surface of the body portion matches the shape of the channel so that it forms an interference fit with the channel; and
   - the upper surface of the body portion is flat and level with the base around the cavity;
c) sealing a lid material to the base and preferably to part of the upper surface of the body portion so as to seal the cavities; and
d) simultaneously or in either order, forming a detachable part of the base and lid materials, and cutting the base and lid materials to form individual dispensers, or pairs of dispensers, or strips with a plurality of dispensers;
wherein the air inlet(s) and the air outlet(s) abut, and are closed by the base material and / or the lid material in such a way that they are opened when the detachable part is removed.

The process is adapted from the standard process for producing blister strips for dry powder inhalers. It can be implemented with mainly conventional materials and existing production equipment. It therefore provides a straightforward and inexpensive way of manufacturing simple unit dose dispensers.

### Brief Description of the Figures

Figure 1 shows a conventional process for producing blister strips for dry powder inhalers.
Figure 2 shows the process for producing an inhaler according to the invention.
Figure 3A shows a dispenser produced by the process of Figure 2. Figure 3B is an expanded view of the components of the dispenser. Figure 3C shows the airway its own. Figure 3D is a transverse cross-section through the body portion of the airway. Figure 3E is a longitudinal cross-section through the end of the mouthpiece. Figures 3F and 3G are isometric views which show the dispenser after the detachable part has been torn off. Figure 3H illustrates the air flow through the dispenser during inhalation.
Figures 4A - 4D show a further embodiment a dispenser. Figures 4A and 4B show the airway its own. Figures 4C and 4D show the dispenser after the detachable part has been torn off.
Figures 5A and 5B show a variant of the dispenser of Figure 4, after the detachable part has been torn off.
Figures 6A illustrates the air flow during inhalation for a further embodiment. Figures 6B and 6C are cross-sectional views along A-A and B-B of Figure 6A respectively.
Figure 7 shows yet another embodiment, with baffles in the air outlet passage.
Figures 8A and 8B show an embodiment in which the airway creates a cyclone in the bowl.
Figure 9A shows a pair of dispensers for simultaneous inhalation. Figure 9B is a cutaway side view when the dispensers are folded so as to lie one on top of the other.
Figures 10A and 10B show strips of six dispensers.

### Detailed Description of the invention

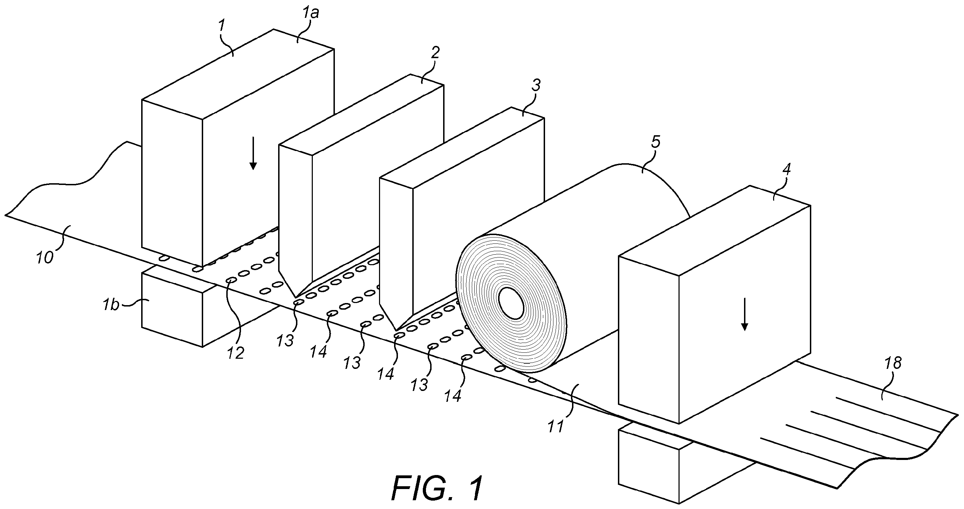

Figure 1 illustrates the conventional process for producing blister strips for dry powder inhalers. The production line has a forming tool **1,** two filling heads **2, 3** and a sealing tool **4.** A sheet of base material **10** passes along the production line from left to right. A roll **5** of lid material **11** is located between the second filling head **3** and the sealing tool **4.**

The base material is typically a laminate comprising a polymer layer in contact with the drug, a soft tempered aluminium foil layer and an external polymer layer, as described for example in WO 2006/108876. The aluminium provides a barrier to ingress of moisture, oxygen and light, whilst the polymer aids the adherence of the foil and provides a relatively inert layer in contact with the drug. Suitable materials for the polymer layer in contact with the drug include polyvinylchloride (PVC), polypropylene (PP) and polyethylene (PE). The polymer layer in contact with the drug is typically PVC of 30µm thickness. However, a thicker or thinner layer of e.g. 60µm or 15µm may be used where a stiffer or more flexible laminate is required. Soft tempered aluminium is ductile so that it can be cold-formed into a blister shape. It is typically 45µm thick. The external polymer layer provides additional strength and toughness to the laminate, and is typically made from oriented polyamide (oPA), typically 25µm thick.

The lid material is typically a foil or a foil laminate preferably comprising a heat seal lacquer, a hard rolled aluminium layer and a top layer of primer, as described for example in WO 2006/108876. The heat seal lacquer bonds to the drug-contacting polymer layer of the base laminate during sealing to provide a seal around the top of the cavity. If the polymer layer in contact with the drug in the base material is PE, the heat seal lacquer on the lid material may be replaced with a further layer of PE. On heat-sealing, the two layers of PE melt and weld to each other. The aluminium layer is typically hard rolled and 20-30 µm thick. The primer facilitates printing onto the strip, for example dose numbers.

The sheet of base material **10** first passes through the forming tool where it is cold formed to create rows of blister cavities **12** by moving the upper part **1a** of the forming tool **1** downwards so that the base material is pressed between the upper **1a** and the lower **1b** parts. Then the formed base sheet passes under the filling heads **2, 3.** Each filling head dispenses measured amounts of powder into a row of cavities. The two filling heads are spaced apart by an odd number of blister pitches (i.e. the distance between the centres of adjacent blister cavities in the longitudinal direction of the base sheet), and the base sheet is advanced by two blister pitches in each step. Thus the first filling head fills odd numbered rows **13** and the second filling head fills even numbered rows **14** of blister cavities. In practice, there may be a larger number of filling heads, for example six, in which case the base sheet advances by six blister pitches in each step. Next, the lid material **11** is dispensed from the roll **5** on top of the base sheet and the sealing tool **4** heats and compresses the base and lid material together in a region surrounding each cavity to form a heat-seal. Knives (not shown) cut the formed, filled and sealed blister sheet longitudinally into blister strips **18** as it advances, and also transversely to the required length.

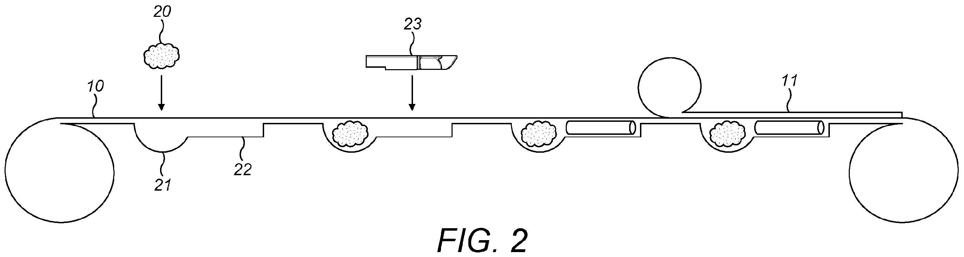

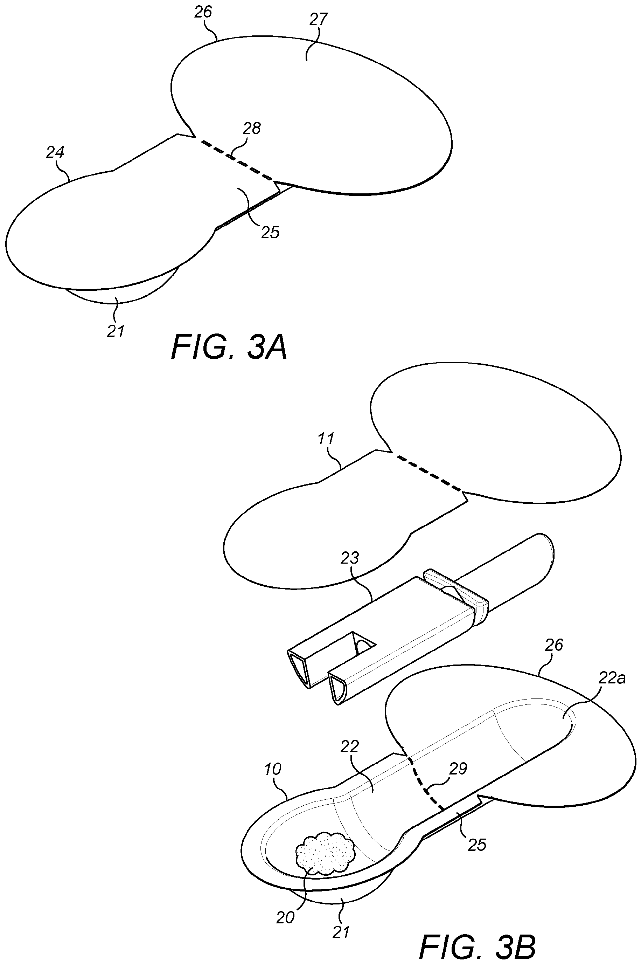

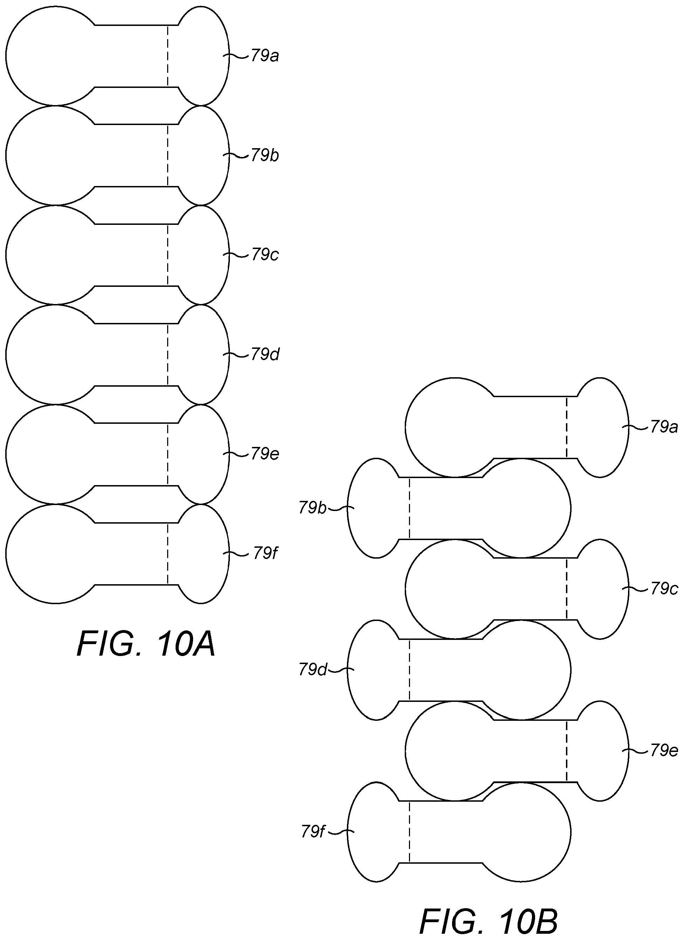

Figure 2 illustrates the process of the invention. The cavities are formed in the base sheet **10** in the same manner as the conventional process, but are shaped differently. As well as a bowl **21** into which the powder **20** is dispensed, the cavities also have a channel **22,** one end of which opens into the bowl. An airway **23** is placed into the channel **22** from a magazine. Although Figure 2 shows the airway **23** being placed into the channel **22** after the powder **20** has been filled into the bowl **21,** these steps may occur simultaneously, or in either order. Placing the airway **23** into the channel **22** before the powder **20** is filled into the bowl **21** has the advantage that this avoids any possibility of powder being trapped underneath the airway. Then the cavities are sealed with lid material **11** in the same manner as the conventional process. The lid material may also bond to part of the upper surface of the airway. Finally, the sheet is cut into individual dispensers, or strips of dispensers. The dispensers may be cut out to form a desired shape, for example with a tab **27,** as shown in Figure 3A. A line of weakness **28** between the tab **27** and the remainder of the dispenser may be formed in this step to facilitate removal of the detachable part **26** (which comprises both the base and lid materials). The line of weakness is typically provided by perforations or scores in the lid and / or base material, and / or a notch in one or both edges. The position of the line of weakness may be indicated by a printed line on the on the lid material. Similarly, a line of weakness may be provided between each dispenser in a strip (as shown in Figure 10), so that individual dispensers can be detached as needed.

Since the process is based on and adapted from the standard process for producing blister strips for dry powder inhalers, it can be implemented using mainly conventional materials and existing production equipment. It therefore provides a simple and inexpensive way of manufacturing unit dose dispensers.

Figure 3A shows the blister produced by the process of Figure 2, after it has been cut out from the sheet to form an individual dispenser. The blister has a main part **24** comprising the bowl **21** and a neck **25,** and a detachable part **26** comprising the tab **27.** Between the main part **24** and the detachable part **26** there is a line of perforations **28** in the lid and a corresponding line of perforations **29** in the base.

Figure 3B is an expanded view of the components of the dispenser, namely the base **10,** the powder **20** located in the bowl **21,** the airway **23** which fits in the channel **22,** and the lid **11.** The channel **22** extends from the bowl **21** along the neck **25** and into the detachable part **26.**

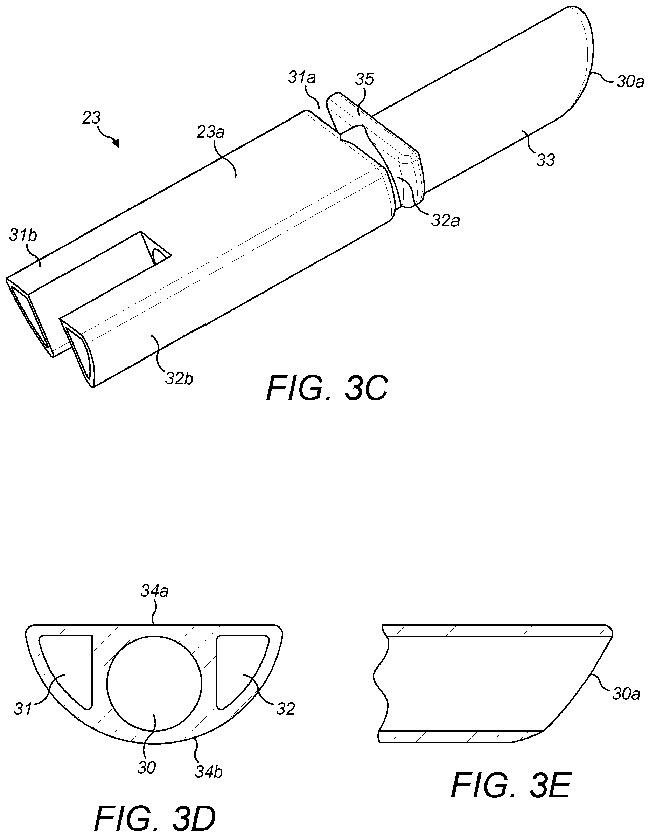

Figure 3C shows the airway **23** which has a body portion **23a.** Figure 3D is a transverse cross-section through the body portion **23a,** which has three passages: a central air outlet passage **30** and two air inlet passages **31, 32,** one on either side. At one end (the proximal end, on the left side in Figure 3C), the air outlet passage **30** and the air inlet passages **31, 32** open into the bowl **21.** The air inlet passages **31, 32** have extensions **31b, 32b** which protrude further into the bowl than the central air outlet passage **30.** At the other end, the air outlet passage is a tube which protrudes beyond the ends of the air inlet passages to form the mouthpiece **33.** The distal end of the air outlet passage, which is shown in longitudinal cross-section in Figure 3E, has an air outlet **30a.** The air inlets **31a, 32a** are formed by the distal ends of the air inlet passages **31, 32** remote from the bowl **21.**

As shown in Figure 3D, the body portion **23a** of the airway **23** has a generally semi-circular cross-section, with a flat upper surface **34a.** The semi-circular lower surface **34b** corresponds in size and shape to the channel **22** so that it is held in the channel by an interference fit. When the airway is located in the channel, the flat upper surface **34a** is level with the base around the cavity (i.e. with the top of the channel). This has the advantage that the lid material of the main part **24** can be sealed to the flat upper surface of the body portion of the airway, as well to the region of the base that surrounds the cavity. This ensures that the airway **23** is held securely in place in the dispenser. The lid material in the detachable part **26** however is not sealed to the upper flat surface of the airway, in order that the detachable part can be detached easily. The airway may accordingly have a heat seal lacquer on the flat upper surface **34a** or be made from PE to facilitate formation of a heat seal with the lid, in the same manner as described above for the base material.

Since the lid material is sealed to the upper flat surface **34a** of the airway **23,** and the lower semi-circular surface **34b** of the airway **23** forms an interference fit with the channel **22,** the bowl forms a closed powder chamber, apart from the air inlet passages **31, 32** and the air outlet passage **30.** Powder could be transferred from the bowl **21** into the air inlet and air outlet passages, and hence into the detachable part between manufacture and use, for example during transport or storage. Any powder that remains within the air inlet and outlet sections of the airway will be aerosolized when the user inhales. However, any powder that enters the detachable part would be lost when it is removed, in which case the full dose would not be delivered.

In order to prevent transfer of powder into the detachable part, a barrier is located a small distance in front of the air inlets **31a, 32a.** The barrier is in the form of a semi-circular wall **35** which forms an interference fit with the channel. Consequently, any powder that is in the air inlet passages **31,32** can leave through the air inlets **31a, 32a,** but cannot get past the wall **35** and so is not able to enter the detachable part **26.** Also, as shown in Figure 3E, the air outlet **30a** is curved so that it matches the shape of the end of the channel **22a.** Since the curved air outlet **30a** abuts the end of the channel **22a,** any powder that is in the air outlet passage **30** cannot exit through the air outlet **30a.** Together with the wall **35,** this ensures that essentially the whole dose is retained within the bowl **21** and the airway **23.** Only powder that is stuck to the parts of the lid adjacent to the air inlets or to the part of the channel that abuts the air outlet could be transferred to the detachable part. The materials from which the lid and base are made are chosen to minimize powder adhesion. As a result, little or no powder is lost when the detachable part is removed, and essentially the whole dose is inhaled. Consequently, in contrast to the devices of US 2017/0119982 and US 2013/0291865, there is no need for a separate way of sealing the powder compartment.

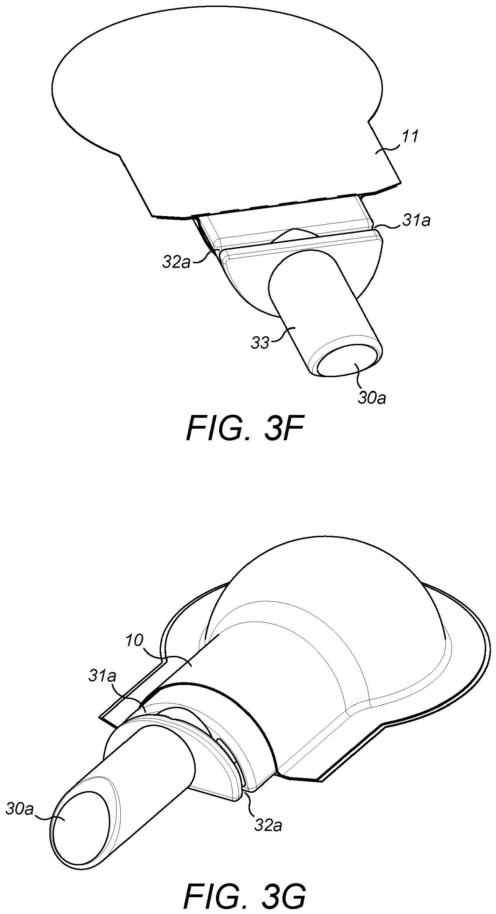

To prepare the dispenser for delivering a dose of medication, the user pulls the tab **27** along the lines of perforations **28, 29** to remove the detachable part **26.** Figures 3F and 3G are isometric views which show the upper and lower sides of the dispenser respectively, after the detachable part **26** has been torn off in order to expose the air inlets **31a, 32a** and the air outlet **30a.** Since the channel **22** and airway **23** extend into the detachable part **26** when it is present, the air inlet passages **31, 32** and the air outlet passage **30** protrude out of the main part **24** which remains after the detachable part **26** has been removed. This ensures that any loose parts of the torn lid material cannot block the air inlets.

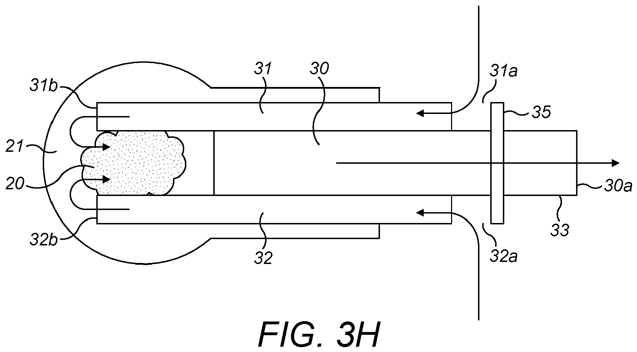

Once the detachable part has been removed, the dispenser is ready to use. The user inhales on the mouthpiece **33.** Since the mouthpiece **33** extends beyond the air inlets **31a, 32a** and the wall **35,** there is no danger of the user's lips blocking the air inlets. Figure 3H illustrates the air flow during inhalation. Air flows into the air inlets **31a, 32a,** through the air inlet passages **31, 32** and enters the bowl **21.** The extensions **31b, 32b** of the air inlet passages **31, 32** which protrude further into the bowl than the air outlet passage **30** ensure that the air flows through the centre of the bowl and aerosolizes the powder **20.** The aerosolized powder then flows into the air outlet passage **30,** along the mouthpiece **33,** through the air outlet **30a** and into the user's lungs.

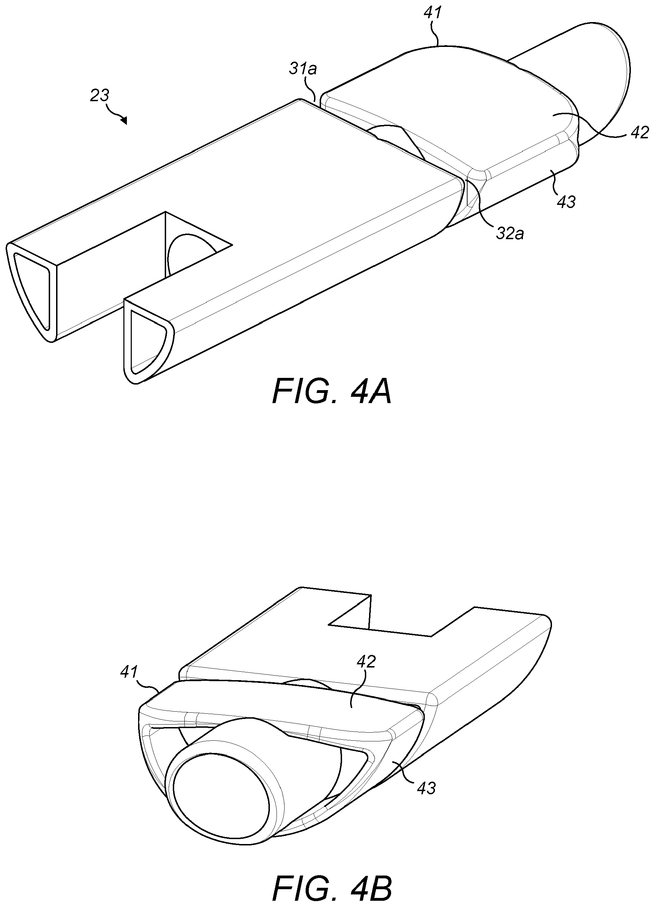

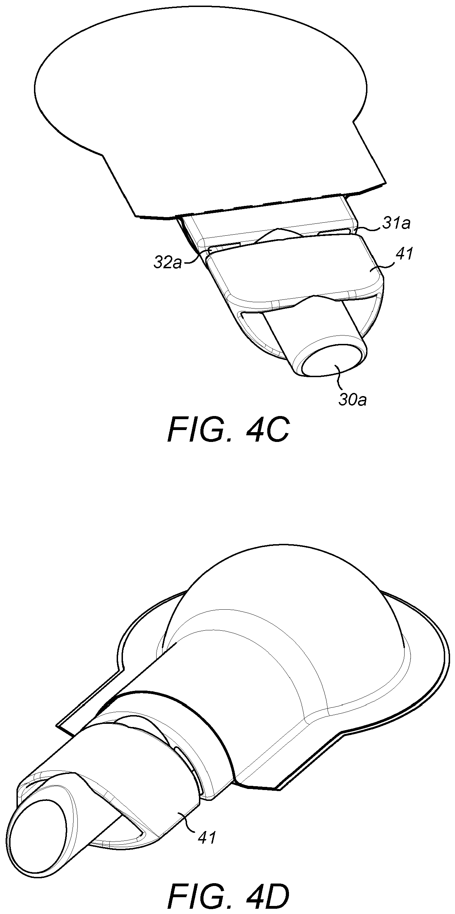

Figures 4A and 4B show an alternative embodiment of the airway **23** in which the barrier is in the form of a block **41** with a semi-circular cross-section which matches the size and shape of the channel. The block **41** is shown in Figure 4B as being a hollow extension from a wall (as in Figure 3), but it could alternatively be solid. Having a hollow or solid block **41** instead of the wall has the advantage that it is even less likely that powder could get past the barrier and into the detachable part. This is because there is close contact between the flat upper surface **42** of the block **41** and the lid, and between the semi-circular lower surface **43** of the block **41** and the channel, over a longer distance. The lid is not sealed to the upper flat surface **42** of the block **41** since doing so would make it difficult to remove the detachable part. Figures 4C and 4D are isometric views which show the upper and lower sides of the dispenser after the detachable part has been torn off so that the air inlets **31a, 32a** and the air outlet **30a** have been exposed. As with the embodiment of Figure 3, the perforations are spaced apart from, and closer to the bowl than, the air inlets **31a, 32a** so that any loose parts of the torn lid material cannot block the air inlets.

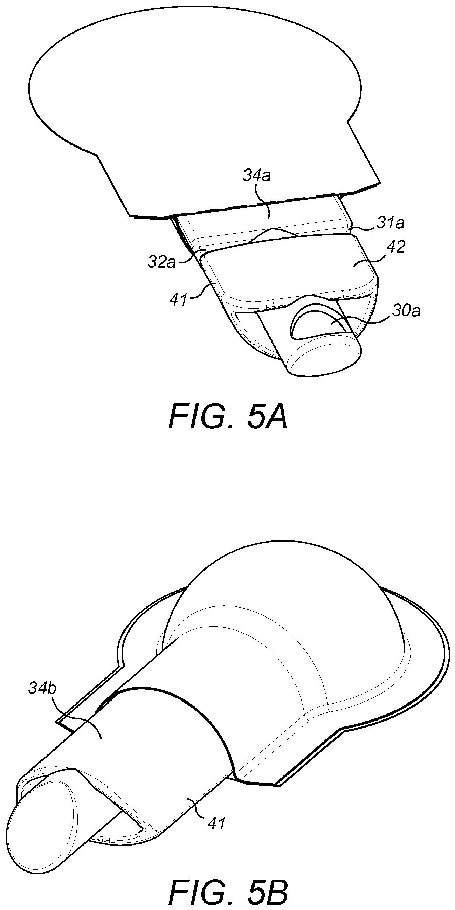

Figures 5A & 5B show a variant of the embodiment of Figure 4, in which the lower surface **34b** of the body portion of the airway extends continuously to the block **41.** Consequently, the air inlets **31a, 32a** are formed only by the gap between the upper flat surface **34a** of the body portion and the upper surface **42** of the block **41.** The air outlet **30a** is formed in the upper surface of the mouthpiece **33** instead of the curved end. Thus both the air outlet **30a** and the air inlets **31a, 32a** abut, and are closed by, the lid. The lid prevents powder from leaving the airway and entering the detachable part, thereby ensuring that the full dose is delivered when the used inhales.

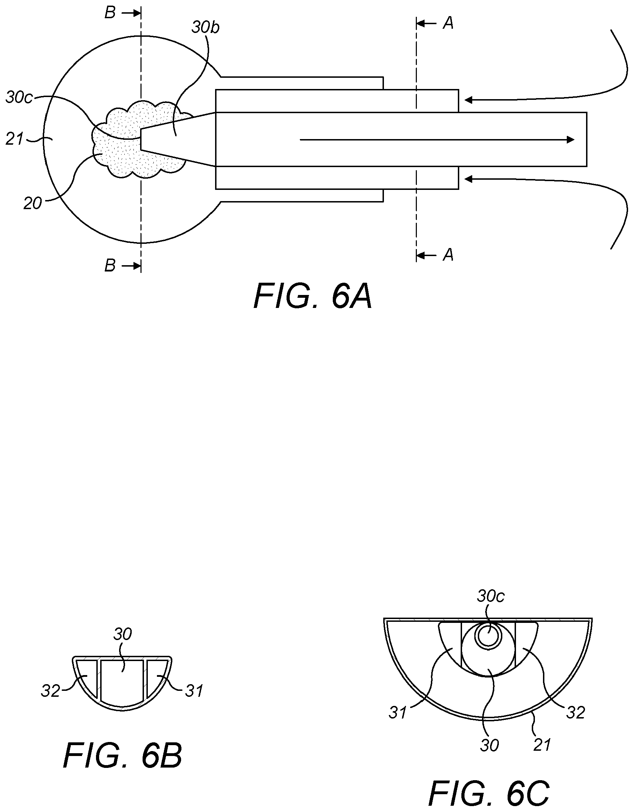

Figures 6A, 6B & 6C show a further embodiment. Figure 6A is a view from above of a dispenser after the detachable part has been removed, but with the lid not shown so that the airway and powder are visible. Figures 6B and 6C are cross-sectional views along A-A and B-B of Figure 6A respectively. The air outlet passage **30** has an extension **30b** that tapers as it protrudes into the bowl **21,** so that the narrow end **30c** of the extension is close to the centre of the bowl. Since the area of the air outlet passage at the point where the powder could enter it is smaller, powder transfer from the bowl into the air outlet passage before use is reduced. The tapering extension may be present in any of the embodiments, for example it may be used in combination with air inlet passages that have extensions which also protrude into the bowl, as in the embodiments of Figures 3 and 4.

The central part of the airways and channels shown in Figures 3 to 6 have a semi-circular cross-section, and the airway has three passages that provide the air inlets and the air outlet. However, the airway and channel may have other cross-sectional shapes, provided that they match, i.e. the airway fits snugly into the channel. The airway could have other numbers of passages, provided that there is at least one air inlet and at least one air outlet. The mouthpiece could have a different shape, such as an elliptical rather than circular cross-section. The extensions of the air inlet passages are shown in Figures 3 and 4 as being straight, but they could alternatively be curved so that they match the inner surface of the bowl; this can help to hold the airway in place so that it cannot be pulled out along the channel.

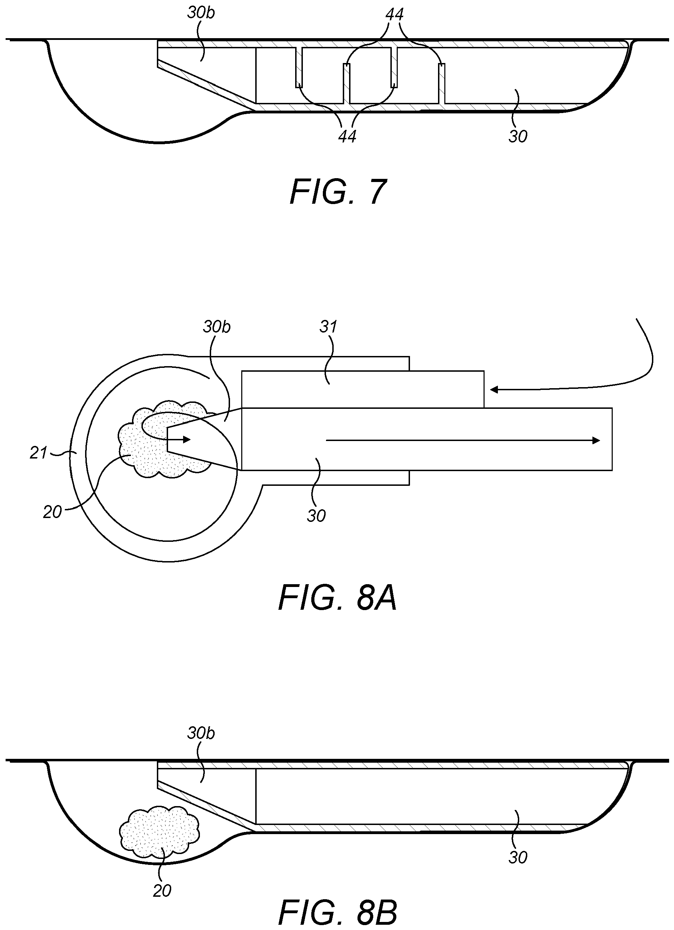

Figure 7 shows a longitudinal cross-section through a dispenser in which the air outlet passage **30** has internal baffles **44.** The baffles help to retain powder that has entered the air outlet passage from the bowl before use. The air inlet passages may similarly have baffles. Additionally or alternatively, the air inlet and / or air outlet passages may have corners or be formed as a labyrinth for reducing the powder transfer through the passages into the detachable part. The baffles / corners / labyrinth may be present in the air inlet and / or outlet passages in any of the embodiments.

Figures 8A and 8B show an embodiment in which the airway is designed to create a cyclone in the bowl. Figure 8A is a view from above of a dispenser after the detachable part has been removed, but with the lid not shown so that the airway and powder are visible. Figure 8B is a longitudinal cross-sectional view through the centre of the dispenser. The airway has an air outlet passage **30** on one side and a single air inlet passage **31** on the other side. This asymmetric arrangement of the air inlet and air outlet passages creates cyclonic motion of the air within the bowl **21** when the user inhales, as indicated by the arrow. The cyclone helps to entrain the powder **20,** and results in the smallest particles circulating near the centre of the bowl, whereas larger particles gravitate towards the edge of the bowl. Since the aerosolized powder enters the air outlet **30** from the top of the centre of the bowl through the extension of the air outlet passage **30b,** the small particles are preferentially delivered to the user's lungs.

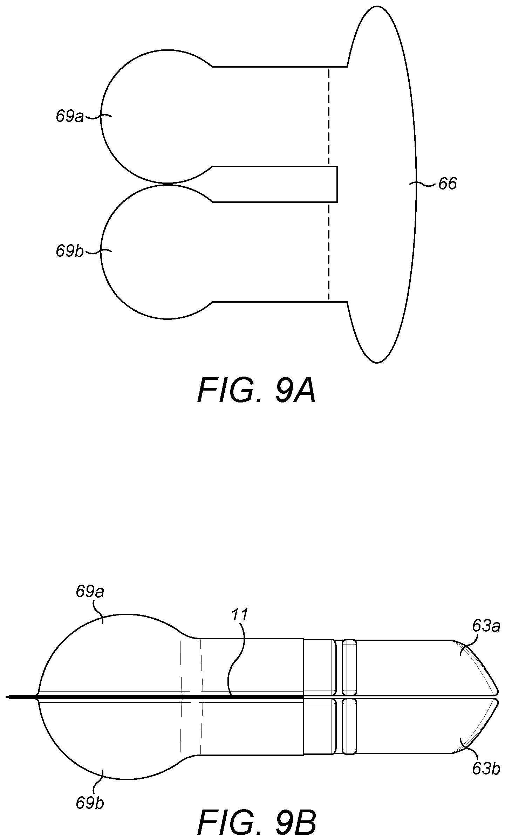

Figure 9 shows an embodiment which is designed to deliver the contents of two dispensers simultaneously, for example in order to deliver a double dose, or to deliver two different medicaments simultaneously, for example if the two medicaments cannot be stored together in a single blister. Figure 9A shows two dispensers **69a, 69b** joined together; the dispensers could contain any of airways described above. The dispensers are cut out as a joined pair, rather than individual dispensers. The user tears off the detachable part **66,** thereby exposing both of the air outlets, which are close enough together that the user can put both of them between their lips and inhale. The dispensers **69a, 69b** could also be folded, for example, after the detachable part has been removed, so that one lies on top of the other (i.e. with the lids **11** touching each other). The air outlets **63a, 63b** are thereby arranged one above the other, as shown in the side view of Figure 9B.

The dispensers may be provided as a strip of several (e.g. six or ten) dispensers **79a-f,** shown in Figure 10. In Figure 10A, the dispensers are all oriented in the same direction; in Figure 10B, adjacent dispensers are oriented in opposite directions. Since in the embodiments shown the main part of the dispenser is wider than the neck, this alternating arrangement allows for closer packing. Each dispenser is detachable from the rest of the strip, for example by a line of perforations, so that individual dispensers can be detached as needed. A number of strips of dispensers may be provided in a pack, for example 30 days' supply of drug in the form of five strips with six dispensers per strip.

The medicament is suitable for administration by inhalation, for example for the treatment of a respiratory disease. It may include one of more of the following classes of pharmaceutically active material: anticholinergics, adenosine A2A receptor agonists, β2-agonists, calcium blockers, IL-13 inhibitors, phosphodiesterase-4-inhibitors, kinase inhibitors, steroids, CXCR2, proteins, peptides, immunoglobulins such as Anti-IG-E, nucleic acids in particular DNA and RNA, monoclonal antibodies, small molecule inhibitors and leukotriene B4 antagonists. The medicament may include excipients, such as fine excipients and / or carrier particles (for example lactose), and / or additives (such as magnesium stearate, phospholipid or leucine).

Suitable β2-agonists include albuterol (salbutamol), e.g. albuterol sulfate; carmoterol, e.g. carmoterol hydrochloride; fenoterol; formoterol; milveterol, e.g. milveterol hydrochloride; metaproterenol, e.g. metaproterenol sulfate; olodaterol; procaterol; salmeterol, e.g. salmeterol xinafoate; terbutaline, e.g. terbutaline sulphate; vilanterol, e.g. vilanterol trifenatate or indacaterol, e.g. indacaterol maleate. Suitable steroids include budesonide; beclamethasone, e.g. beclomethasone dipropionate; ciclesonide; fluticasone, e.g. fluticasone furoate; mometasone, e.g. mometasone furoate. Suitable anticholinergics include: aclidinium, e.g. aclidinium bromide; glycopyrronium, e.g. glycopyrronium bromide; ipratropium, e.g. ipratropium bromide; oxitropium, e.g. oxitropium bromide; tiotropium, e.g. tiotropium bromide; umeclidinium, e.g. umeclidinium bromide; Darotropium bromide; or tarafenacin.

The active material may include double or triple combinations such as salmeterol xinafoate and fluticasone propionate; budesonide and formoterol fumarate dihydrate glycopyrrolate and indacaterol maleate; glycopyrrolate, indacaterol maleate and mometasone furoate; fluticasone furoate and vilanterol; vilanterol and umeclidinium bromide; fluticasone furoate, vilanterol and umeclidinium bromide.

The invention provides a very simple unit dose dry powder inhaler. It can be manufactured using an adapted version of the conventional process for producing blisters, and only one simple additional component (the airway) is required. Consequently, the dispenser is inexpensive and easy to produce.

## Claims

1. A unit dose dispenser in the form of a blister containing a dry powder for inhalation, the dispenser comprising:
• a base (10) in which a cavity is formed, wherein the cavity comprises a bowl (21) that contains the powder (20) and a channel (22) that opens into the bowl, and has an end remote from the bowl;
• an airway (23) located in the channel, the airway comprising a mouthpiece (33) and a body portion (23a) having at least one air outlet passage (30), at least one air inlet passage (31,32), a lower surface (34b) which forms an interference fit with the channel, and an upper surface (34a) which is flat and level with the base around the cavity; and
• a lid (11) which is sealed to the base around the cavity, and preferably to part of the upper surface of the body portion;
wherein:
• each air outlet passage opens into the bowl at a proximal end and extends from the body portion through the mouthpiece to an air outlet (30a) at a distal end;
• each air inlet passage opens into the bowl at a proximal end and has an air inlet (31a, 32a) at a distal end;
• the air inlet(s) and the air outlet(s) abut and are closed by the base and / or the lid; and
• part (26) of the base and lid of the dispenser is detachable, so that the air inlet(s) and the air outlet(s) are opened when the detachable part is removed.

2. A unit dose dispenser according to claim 1, wherein the body portion and the channel are semi-circular in cross-section.

3. A unit dose dispenser according to claim 1 or claim 2, wherein the air outlet(s) match the shape of, and are closed by the end of the channel.

4. A unit dose dispenser according to claim 1 or claim 2, wherein the air outlet(s) are formed in the upper surface of the mouthpiece and are closed by the lid.

5. A unit dose dispenser according to any of claims 1 to 4, wherein the airway comprises a barrier located between the air inlet(s) and the air outlet(s), wherein the size and shape of the barrier corresponds to the cross-section of the channel.

6. A unit dose dispenser according to claim 5, wherein the barrier is a wall (35) or a block (41).

7. A unit dose dispenser according to claim 5 or claim 6, wherein the lower surface of the body portion extends continuously to the barrier so that the air inlet(s) are formed in the upper surface of the airway and are closed by the lid.

8. A unit dose dispenser according to any of claims 1 to 7, wherein the air inlet and / or air outlet passages have baffles (44) or corners or are formed as a labyrinth.

9. A unit dose dispenser according to any of claims 1 to 8, wherein the airway has one air outlet passage (30), preferably with an extension (30b) that protrudes into the bowl (21) and which tapers to a narrow end (30c) located at, or close to, the centre of the bowl.

10. A unit dose dispenser according to any of claims 1 to 9, wherein the airway has one air outlet passage and one air inlet passage that cause an asymmetric air flow which creates a cyclone in the bowl.

11. A unit dose dispenser according to any of claims 1 to 9, wherein the airway has one air outlet passage (30) and two air inlet passages (31,32), one on either side of the air outlet passage, which preferably have extensions (31b, 32b) that protrude into the bowl (21).

12. A unit dose dispenser according to any of claims 1 to 11 which has a line of weakness (28, 29) in the base and / or the lid, and / or a notch in one or both edges of the base and / or the lid.

13. A pair of dispensers (69a, 69b) according to any of claims 1 to 12 which are joined together so that the powders in both dispensers can be inhaled simultaneously.

14. A strip comprising a plurality of dispensers (79a - 79f) according to any of claims 1 to 12 wherein each dispenser is detachable from the rest of the strip.

15. A process for producing unit dose dispensers in the form of blisters containing a dry powder for inhalation, the process comprising:
a) forming cavities in a base material (10), each cavity comprising a bowl (21), and a channel (22) that opens into the bowl and has an end remote from the bowl;
b) simultaneously or in either order, filling the powder (20) into the bowl and placing an airway (23) into the channel, wherein the airway comprises a mouthpiece (33) and a body portion (23a) having at least one air outlet passage (30), at least one air inlet passage (31, 32), a lower surface (34b) which forms an interference fit with the channel, and an upper surface (34a) which is flat and level with the base material around the cavity;
c) sealing a lid material (11) to the base and preferably to part of the upper surface of the body portion of the airway; and
d) simultaneously or in either order, forming a detachable part (26) of the base and lid materials and cutting the base and lid materials to form individual dispensers, or pairs of dispensers (69a, 69b), or strips with a plurality of dispensers (79a - 79f);
wherein
• each air outlet passage opens into the bowl at a proximal end and extends from the body portion through the mouthpiece to an air outlet (30a) at a distal end;
• each air inlet passage opens into the bowl at a proximal end and has an air inlet (31a, 32a) at a distal end; and
• the air inlet(s) and the air outlet(s) abut and are closed by the base material and / or the lid material in such a way that they are opened when the detachable part is removed.

## Patentansprüche

1. Einheitsdosisspender in der Form eines Blisters, der ein Trockenpulver zur Inhalation enthält, wobei der Spender Folgendes umfasst:
• eine Basis (10), in der eine Vertiefung ausgebildet ist, wobei die Vertiefung eine Schale (21), die das Pulver (20) enthält, und einen Kanal (22), der in die Schale mündet und ein von der Schale abgelegenes Ende hat, umfasst,
• einen in dem Kanal angeordneten Luftweg (23), wobei der Luftweg ein Mundstück (33) und einen Körperabschnitt (23a) umfasst, der mindestens einen Luftauslassdurchgang (30), mindestens einen Lufteinlassdurchgang (31, 32), eine untere Fläche (34b), die eine Presspassung mit dem Kanal bildet, und eine obere Fläche (34a) hat, die flach ist und mit der Basis um die Vertiefung herum auf gleicher Höhe liegt, und
• einen Deckel (11), der mit der Basis um die Vertiefung herum und vorzugsweise mit einem Teil der oberen Fläche des Körperabschnitts versiegelt ist, wobei:
• jeder Luftauslassdurchgang an einem proximalen Ende in die Schale mündet und sich von dem Körperabschnitt durch das Mundstück zu einem Luftauslass (30a) an einem distalen Ende erstreckt,
• jeder Lufteinlassdurchgang an einem proximalen Ende in die Schale mündet und einen Lufteinlass (31a, 32a) an einem distalen Ende hat,
• der/die Lufteinlass/-einlässe und der/die Luftauslass/-auslässe an der Basis und/oder dem Deckel anliegen und von der Basis und/oder dem Deckel verschlossen werden, und
• Teil (26) der Basis und des Deckels des Spenders abtrennbar ist, so dass der/die Lufteinlass/-einlässe und der/die Luftauslass/-auslässe geöffnet werden, wenn der abtrennbare Teil entfernt wird.

2. Einheitsdosisspender nach Anspruch 1, wobei der Körperabschnitt und der Kanal einen halbkreisförmigen Querschnitt haben.

3. Einheitsdosisspender nach Anspruch 1 oder Anspruch 2, wobei der/die Luftauslass/-auslässe der Form des Endes des Kanals entspricht/entsprechen und von diesem verschlossen wird/werden.

4. Einheitsdosisspender nach Anspruch 1 oder Anspruch 2, wobei der/die Luftauslass/-auslässe in der oberen Fläche des Mundstücks ausgebildet ist/sind und von dem Deckel verschlossen wird/werden.

5. Einheitsdosisspender nach einem der Ansprüche 1 bis 4, wobei der Luftweg eine Sperre umfasst, die zwischen dem/den Lufteinlass/-einlässen und dem/den Luftauslass/-auslässen angeordnet ist, wobei die Größe und Form der Sperre dem Querschnitt des Kanals entspricht.

6. Einheitsdosisspender nach Anspruch 5, wobei die Sperre eine Wand (35) oder ein Block (41) ist.

7. Einheitsdosisspender nach Anspruch 5 oder Anspruch 6, wobei sich die untere Fläche des Körperabschnitts kontinuierlich zu der Sperre erstreckt, so dass der/die Lufteinlass/-einlässe in der oberen Fläche des Luftwegs ausgebildet ist/sind und von dem Deckel verschlossen wird/werden.

8. Einheitsdosisspender nach einem der Ansprüche 1 bis 7, wobei die Lufteinlass- und/oder Luftauslassdurchgänge Schikanen (44) oder Ecken haben oder als ein Labyrinth ausgebildet sind.

9. Einheitsdosisspender nach einem der Ansprüche 1 bis 8, wobei der Luftweg einen Luftauslassdurchgang (30), vorzugsweise mit einer Verlängerung (30b) hat, die in die Schale (21) ragt und die sich zu einem schmalen Ende (30c) verjüngt, das in der oder in der Nähe der Mitte der Schale angeordnet ist.

10. Einheitsdosisspender nach einem der Ansprüche 1 bis 9, wobei der Luftweg einen Luftauslassdurchgang und einen Lufteinlassdurchgang hat, die eine asymmetrische Luftströmung veranlassen, die eine Zyklonwirkung in der Schale erzeugt.

11. Einheitsdosisspender nach einem der Ansprüche 1 bis 9, wobei der Luftweg einen Luftauslassdurchgang (30) und zwei Lufteinlassdurchgänge (31, 32), und zwar einen auf jeder Seite des Luftauslassdurchgangs, aufweist, die vorzugsweise Verlängerungen (31b, 32b) haben, die in die Schale (21) ragen.

12. Einheitsdosisspender nach einem der Ansprüche 1 bis 11, der eine Schwächungslinie (28, 29) in der Basis und/oder dem Deckel und/oder eine Kerbe in einem oder beiden Rändern der Basis und/oder des Deckels hat.

13. Spenderpaar (69a, 69b) nach einem der Ansprüche 1 bis 12, die miteinander verbunden sind, so dass die Pulver in beiden Spendern gleichzeitig inhaliert werden können.

14. Streifen, umfassend eine Vielzahl von Spendern (79a - 79f) nach einem der Ansprüche 1 bis 12, wobei jeder Spender von dem Rest des Streifens ablösbar ist.

15. Verfahren zur Herstellung von Einheitsdosisspendern in der Form von Blistern, die ein Trockenpulver zur Inhalation enthalten, wobei das Verfahren Folgendes umfasst:
a) Ausbilden von Vertiefungen in einem Basismaterial (10), wobei jede Vertiefung eine Schale (21) und einen Kanal (22) umfasst, der in die Schale mündet und ein von der Schale abgelegenes Ende hat,
b) Füllen des Pulvers (20) in die Schale und Platzieren eines Luftwegs (23) in den Kanal, und zwar gleichzeitig oder in beliebiger Abfolge, wobei der Luftweg ein Mundstück (33) und einen Körperabschnitt (23a) umfasst, der mindestens einen Luftauslassdurchgang (30), mindestens einen Lufteinlassdurchgang (31, 32), eine untere Fläche (34b), die eine Presspassung mit dem Kanal bildet, und eine obere Fläche (34a) hat, die flach ist und mit dem Basismaterial um die Vertiefung herum auf gleicher Höhe liegt,
c) Versiegeln eines Deckelmaterials (11) mit der Basis und vorzugsweise einem Teil der oberen Fläche des Körperabschnitts des Luftwegs und
d) Ausbilden eines abtrennbaren Teils (26) des Basis- und des Deckelmaterials und Schneiden des Basis- und des Deckelmaterials zum Ausbilden von einzelnen Spendern oder Spenderpaaren (69a, 69b) oder von Streifen mit einer Vielzahl von Spendern (79a - 79f), und zwar gleichzeitig oder in beliebiger Abfolge,
wobei
• jeder Luftauslassdurchgang an einem proximalen Ende in die Schale mündet und sich von dem Körperabschnitt durch das Mundstück zu einem Luftauslass (30a) an einem distalen Ende erstreckt,
• jeder Lufteinlassdurchgang an einem proximalen Ende in die Schale mündet und einen Lufteinlass (31a, 32a) an einem distalen Ende hat, und
• der/die Lufteinlass/-einlässe und der/die Luftauslass/-auslässe an dem Basismaterial und/oder dem Deckelmaterial anliegen und von dem Basismaterial und/oder dem Deckelmaterial verschlossen werden, so dass sie geöffnet werden, wenn der abtrennbare Teil entfernt wird.

## Revendications

1. Distributeur de dose unitaire sous forme de blister contenant une poudre sèche pour inhalation, le distributeur comprenant :
• une base (10) dans laquelle est formée une cavité, la cavité comprenant un bol (21) qui contient la poudre (20) et un canal (22) qui s'ouvre dans le bol et comporte une extrémité éloignée du bol ;
• une voie aérienne (23) située dans le canal, la voie aérienne comprenant un embout buccal (33) et une partie corps (23a) comportant au moins un passage de sortie d'air (30), au moins un passage d'entrée d'air (31, 32), une surface inférieure (34b) qui forme un ajustement serré avec le canal, et une surface supérieure (34a) qui est plate et de niveau avec la base autour de la cavité ; et
• un couvercle (11) qui est scellé à la base autour de la cavité, et de préférence à une partie de la surface supérieure de la partie corps ;
• chaque passage de sortie d'air s'ouvrant dans le bol au niveau d'une extrémité proximale et s'étendant à partir de la partie corps à travers l'embout buccal jusqu'à une sortie d'air (30a) au niveau d'une extrémité distale ;
• chaque passage d'entrée d'air s'ouvrant dans le bol au niveau d'une extrémité proximale et possédant une entrée d'air (31a, 32a) au niveau d'une extrémité distale ;
• la ou les entrées d'air et la ou les sorties d'air venant en butée et étant fermées par la base et/ou le couvercle ; et
• une partie (26) de la base et du couvercle du distributeur étant amovible, de sorte que la ou les entrées d'air et la ou les sorties d'air soient ouvertes lorsque la partie amovible est retirée.

2. Distributeur de dose unitaire selon la revendication 1, la partie corps et le canal présentant une section transversale semi-circulaire.

3. Distributeur de dose unitaire selon la revendication 1 ou la revendication 2, la ou les sorties d'air correspondant à la forme de l'extrémité du canal et étant fermées par celle-ci.

4. Distributeur de dose unitaire selon la revendication 1 ou la revendication 2, la ou les sorties d'air étant formées dans la surface supérieure de l'embout buccal et étant fermées par le couvercle.

5. Distributeur de dose unitaire selon l'une quelconque des revendications 1 à 4, la voie aérienne comprenant une barrière située entre la ou les entrées d'air et la ou les sorties d'air, la taille et la forme de la barrière correspondant à la section transversale du canal.

6. Distributeur de dose unitaire selon la revendication 5, la barrière étant un mur (35) ou un bloc (41).

7. Distributeur de dose unitaire selon la revendication 5 ou la revendication 6, la surface inférieure de la partie corps s'étendant en continu jusqu'à la barrière de sorte que la ou les entrées d'air soient formées dans la surface supérieure de la voie aérienne et soient fermées par le couvercle.

8. Distributeur de dose unitaire selon l'une quelconque des revendications 1 à 7, les passages d'entrée d'air et/ou de sortie d'air comportant des chicanes (44) ou des coins ou étant formés comme un labyrinthe.

9. Distributeur de dose unitaire selon l'une quelconque des revendications 1 à 8, la voie aérienne comportant un passage de sortie d'air (30), de préférence avec une extension (30b) qui fait saillie dans le bol (21) et qui se rétrécit jusqu'à une extrémité étroite (30c) située au centre du bol ou à proximité de celui-ci.

10. Distributeur de dose unitaire selon l'une quelconque des revendications 1 à 9, la voie aérienne comportant un passage de sortie d'air et un passage d'entrée d'air qui provoquent un flux d'air asymétrique créant un cyclone dans le bol.

11. Distributeur de dose unitaire selon l'une quelconque des revendications 1 à 9, la voie aérienne comportant un passage de sortie d'air (30) et deux passages d'entrée d'air (31, 32), un de chaque côté du passage de sortie d'air, qui comportent de préférence des extensions (31b, 32b) qui font saillie dans le bol (21).

12. Distributeur de dose unitaire selon l'une quelconque des revendications 1 à 11, qui présente une ligne de faiblesse (28, 29) dans la base et/ou le couvercle, et/ou une encoche dans l'un ou les deux bords de la base et/ou du couvercle.

13. Paire de distributeurs (69a, 69b) selon l'une quelconque des revendications 1 à 12, qui sont assemblés de sorte que les poudres contenues dans les deux distributeurs puissent être inhalées simultanément.

14. Bande comprenant une pluralité de distributeurs (79a - 79f) selon l'une quelconque des revendications 1 à 12, chaque distributeur pouvant être retiré du reste de la bande.

15. Procédé de fabrication de distributeurs de dose unitaire sous forme de blisters contenant une poudre sèche pour inhalation, le procédé comprenant les étapes consistant à :
a) former des cavités dans un matériau de base (10), chaque cavité comprenant un bol (21) et un canal (22) qui s'ouvre dans le bol et comporte une extrémité éloignée du bol ;
b) simultanément ou dans l'un ou l'autre ordre, remplir la poudre (20) dans le bol et placer une voie aérienne (23) dans le canal, la voie aérienne comprenant un embout buccal (33) et une partie corps (23a) comportant au moins un passage de sortie d'air (30), au moins un passage d'entrée d'air (31, 32), une surface inférieure (34b) qui forme un ajustement serré avec le canal, et une surface supérieure (34a) qui est plate et de niveau avec le matériau de base autour de la cavité ;
c) sceller un matériau de couvercle (11) à la base et, de préférence, à une partie de la surface supérieure de la partie corps de la voie aérienne ; et
d) simultanément ou dans l'un ou l'autre ordre, former une partie amovible (26) des matériaux de base et de couvercle et couper les matériaux de base et de couvercle pour former des distributeurs individuels, ou des paires de distributeurs (69a, 69b), ou des bandes avec une pluralité de distributeurs (79a - 79f) ;
• chaque passage de sortie d'air s'ouvrant dans le bol au niveau d'une extrémité proximale et s'étendant à partir de la partie corps à travers l'embout buccal jusqu'à une sortie d'air (30a) au niveau d'une extrémité distale ;
• chaque passage d'entrée d'air s'ouvrant dans le bol au niveau d'une extrémité proximale et comportant une entrée d'air (31a, 32a) au niveau d'une extrémité distale ; et
• la ou les entrées d'air et la ou les sorties d'air venant en butée et étant fermées par le matériau de base et/ou le matériau de couvercle de sorte qu'elles s'ouvrent lorsque la partie amovible est retirée.
